# EUROPEAN PATENT APPLICATION

(11) **EP 1 402 829 A2**
(43) Date of publication of application: **31.03.2004**
(21) Application number: 03020809.4
(22) Date of filing: 12.09.2003
(51) Int. Cl.: A61B 17/70

(54) **Vertebra body plate for vertebral column fixing system**

(30) Priority: 12.09.2002 JP 2002267300
(71) Applicant: Showa IKA Kohgyo Co., Ltd., Aichi-ken (JP)
(72) Inventor: Suzuki, Nobumasa, Tokyo (JP); Nohara, Hiroshi, Koshigaya-shi Saitama-ken (JP); Nakahara, Shinnosuke, Okayama-shi Okayama-ken (JP); Sato, Shigenobu, Sapporo-shi Hokkaido (JP); Ueyama, Kazumasa, Hirosaki-shi Aomori-ken (JP); Hasegawa, Kazuhiro, Niigata-shi Niigata-ken (JP); Oribe, Kazuya c/o Showa Ika Kohgyo Co.Ltd., Nagoya-shi Aichi-ken (JP); Takamido, Hiroshi c/o Showa Ika Kohgyo Co.Ltd., Nagoya-shi Aichi-ken (JP)
(74) Representative: Grünecker, Kinkeldey, Stockmair & Schwanhäusser Anwaltssozietät

(57) **Abstract**

A vertebra body plate 1 is comprised of a main body portion 3 having an approximately point symmetric shape around a point O in which a first diagonal line M connecting a pair of acute angle portions A, C' having acute angles opposing to each other, crosses a second diagonal line N connecting a pair of obtuse angle portions B, D' having obtuse angles opposing to each other, a pair of screw insertion holes 9 provided on or near the first diagonal line M of the main body portion 3, and two spike 7 provided on each of the first diagonal line M and the second diagonal line

## Description

### CROSS REFERENCE TO RELATED APPLICATIONS

This application is based upon and claims the benefit of priority from the prior Japanese Patent Applications No. P2002-267300, filed on September 12, 2002; the entire contents of which are incorporated herein by reference.

### BACKGROUND OF THE INVENTION

### 1. Field of the Invention

The present invention relates to a vertebra body plate for a vertebral column fixing system, and more particularly to a vertebra body plate which can be easily placed in a vertebra body.

### 2. Description of the Related Art

As an example of a conventional vertebral column fixing method, there is Japanese Patent Application Laid-Open No. 8-229052. The fixing method according to the related art is comprised of a plurality of spikes inserted into the vertebral column, a pair of square connection devices provided with engagement holes engaging with bolts screwed into the vertebral column and a connection member for connecting the connection devices.

According to the structure, since a rigidity of the connection devices is great, there is a problem that it is hard to place the connection device along a shape of the vertebra body of the vertebral column having a curvature. Further, there is a problem that a contact area between the connection device and the vertebra body is small.

### SUMMARY OF THE INVENTION

The present invention is made by taking the conventional problems mentioned above into consideration. A content of the present invention is a rhombic vertebra body plate for a vertebral column fixing system comprising: a main body portion having an approximately point symmetric shape around a point in which a first diagonal line connecting a pair of acute angle portions having acute angles opposing to each other, crosses a second diagonal line connecting a pair of obtuse angle portions having obtuse angles opposing to each other; at least one screw insertion hole provided on or near the first diagonal line of the main body portion or on or near the second diagonal line; and at least one spike provided on each of the first diagonal line and the second diagonal line.

### BRIEF DESCRIPTION OF THE DRAWINGS

Fig. 1 is a perspective view of a vertebra body plate according to a first embodiment of the present invention;
Fig. 2 is a plan schematic view for explaining a shape of the vertebra body plate according to the first embodiment of the present invention;
Fig. 3 is a cross sectional view of the vertebra body plate according to the first embodiment of the present invention;
Fig. 4 is a bottom elevational view of the vertebra body plate according to the first embodiment of the present invention;
Fig. 5A is a plan view of a vertebra body plate according to a second embodiment of the present invention; and
Fig. 5B is a perspective view of the vertebra body plate according to the second embodiment of the present invention.

### DETAILED DESCRIPTION OF THE INVENTION

### First Embodiment

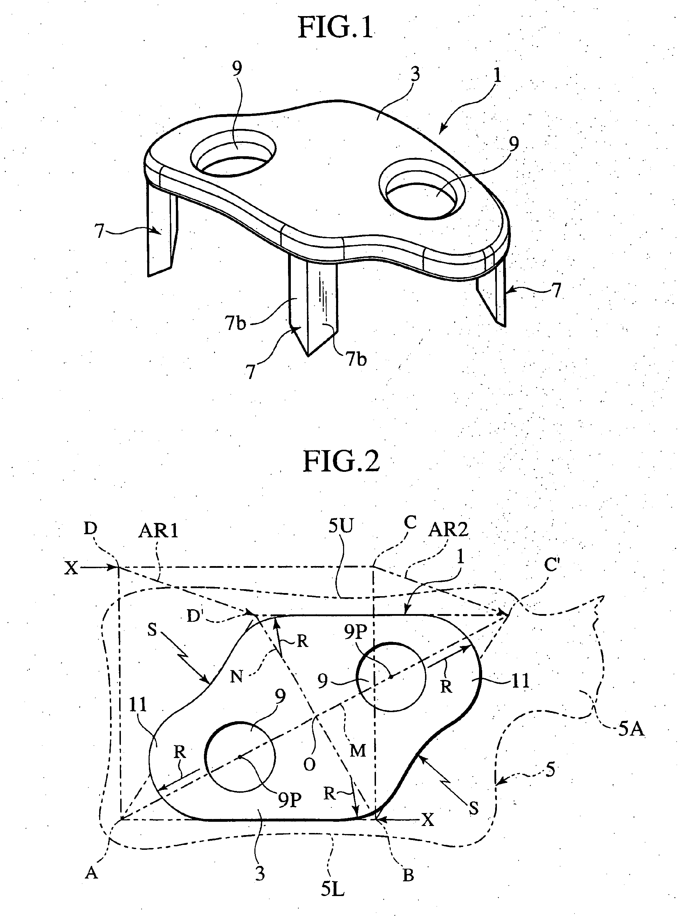

As shown in Figs. 1 and 2, a vertebra body plate 1 according to a first embodiment of the present invention is comprised of a plate main body 3 (a main body portion) made of a material which can be slightly plastically deformed by a great external force, a plurality of spikes 7 provided on the plate main body 3 and stuck into a vertebra body 5 of a vertebral column (refer to Fig. 2), and a plurality of screw insertion holes 9 through which screws (not shown) screwed into the vertebra body 5 are inserted. Further, the spike 7 is in the form of an approximately V-shaped in cross section, and is comprised of a pair of surfaces 7a and 7b opposing to each other. A distal end portion of the spike 7 is tapered and is structured such as to easily stick into the vertebra body.

A shape of the plate main body 3 is formed by the following steps. Firstly, as shown in Fig. 2, when pressing corner portions C and D in a virtual quadrangle ABCD in which four corner portions form 90 degrees in a direction of an arrow X, the corner portions C and D are moved along arrows AR1 and AR2 , and a rhomboid ABC' D' is formed. In the rhomboid ABC'D', the corner portions A and C' oppose to each other and form an acute angle, and the corner portions B and D' oppose to each other and form an obtuse angle . Further, a line AB and a line C'D' form linear edge portions approximately in parallel to upper and lower end plates 5U and 5L in the vertebra body 5, and a line AD' and a line BC ' are inclined with respect to the end plates 5U and 5L . Further, the corner portion C' is positioned in a protruding side (a protruding edge portion) relatively protruding with respect to the line AB.

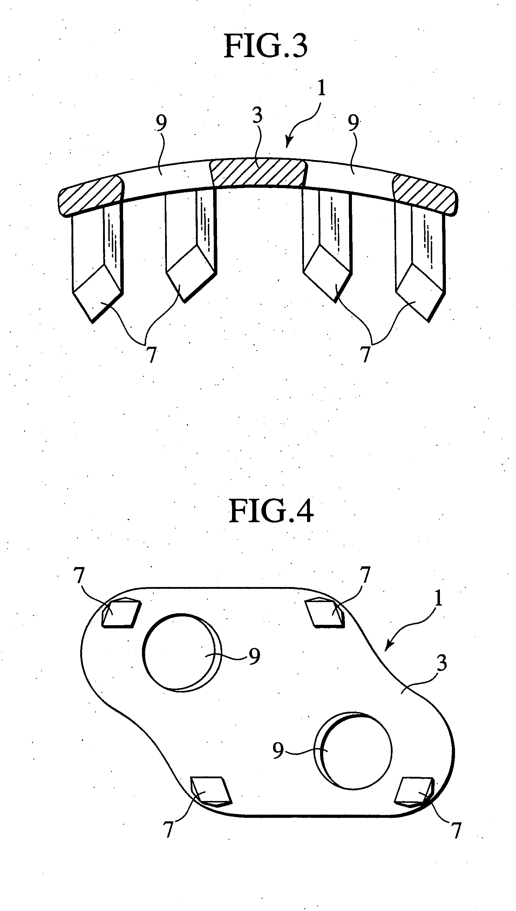

On the assumption that an intersection point between a line M connecting the corner portion A and the corner portion C', and a line N connecting the corner portion B and the corner portion D' is set to O, centers 9P of the screw insertion holes 9 , 9 are provided in symmetrical positions on the line M around the intersection point O. Further, a pair of spikes 7 are provided in symmetrical positions outside the screw insertion holes 9 (refer to Fig. 4). In other words, a distance from the center O to one center 9P (spike 7) is equal to a distance from the center O to another center 9P (spike 7).

In this case, a pair of spikes 7 may be arranged on the line M, or may be slightly displaced from the line M within an allowable range. Further, a pair of spikes 7 are provided in the symmetrical positions on the line N around the intersection point O or the symmetrical position near the line N.

The corner portions A, B, C' and D' of the plate main body 3 have a curvature R. This curvature R is appropriately determined on the basis of the distance from the center 9P of the screw insertion hole 9 to the line AB and the line C' D' and the like. Further, a part of the lines BC' and AD' has a curvature S, and is smoothly connected to the portion of the curvature R. This curvature S is also appropriately determined on the basis of the curvature R and the like. As shown in Fig. 2 , the line D'C' is smoothly connected to the portion having the curvature S from the protruding portion 11 having the curvature R, and is thereafter smoothly connected again to the line AB by the curvature R.

Further, the plate main body 3 is structured, as shown in Fig. 2, such that the upper and lower lines AB and C'D' of the plate main body 3 are approximately in parallel to the upper and lower end plates 5U and 5L of the vertebra body 5, and the corner portion C' corresponding to a pedicle arch vertebra 5A of the vertebra has a shape provided with the protruding portion 11 protruding to a side of the pedicle of arch of the vertebra 5A and an approximately point symmetrical shape around the intersection point O. Further, as shown in Fig. 3, the plate main body 3 is formed in a slightly curved manner so as to correspond to a peripheral surface of the vertebra body 5. In this case, the structure of the plate 3 may be obtained by deforming the point symmetrical shape within the allowable range.

### Second Embodiment

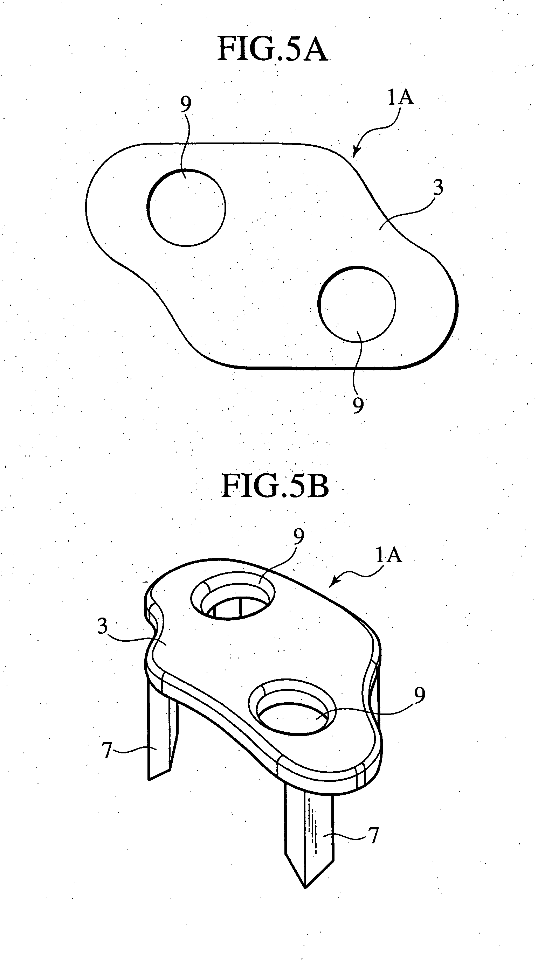

Figs. 5A and 5B show a vertebra body plate 1A having a symmetrical shape with the vertebra body plate 1. A shape thereof is symmetrical with that of the first embodiment, and the other structures are the same as those of the first embodiment mentioned above. Accordingly, the same reference numerals are attached to the constituting portions achieving the same functions, and a detailed description thereof will be omitted.

In the structure mentioned above, the vertebra body plate 1A is used for a vertebral column fixing system in which a plurality of screws (not shown) are screwed into the separated vertebra bodies 5 in the vertebral column, and a portion near an end portion of a rod for connecting the vertebra body is supported and fixed by a rod formed in a head portion of the screw. The vertebra body plate 1A is fixed to the vertebra body 5 before the screw is screwed into the vertebra body 5.

Since the shape of the vertebra body 5 constituting the vertebral column has an individual difference, the vertebra body plate 1A is selected in correspondence to the shape of the individual vertebra body. Further, when placing the vertebra body plate 1 (1A) in the vertebra body 5, the shape of the vertebra body plate 1 is deformed so as to approximately correspond to the surface shape of the vertebra body to be placed.

The vertebra body plate 1 (1A) can be deformed in its shape in correspondence to the curvature of the vertebra body by bending the protruding portions 11, 11 around the center O, and a contact area between the vertebra body plate 1 (1A) and the vertebra body is larger than the conventional one.

Accordingly, since the contact area is large in the case that a plurality of spikes 7 provided in the vertebra body plate 1 (1A) are stuck to the vertebra body 5 so as to be fixed, it is possible to more securely fix the vertebra body plate 1 (1A) to the vertebra body 5.

After respectively fixing the vertebra body plates 1 (1A) to the separated vertebra bodies 5 in the manner mentioned above, the screw (not shown) is screwed into each of the vertebra bodies 5 through the screw insertion hole 9 provided in each of the vertebra body plate 1 (1A), and the portion near the end portion of the rod for connecting the vertebra body is supported and fixed by the rod engagement portion provided in the head portion of the screw, whereby the separated vertebra bodies 5 are connected, and the vertebral column can be fixed.

## Claims

1. A rhombic vertebra body plate for a vertebral column fixing system comprising:
a main body portion having an approximately point symmetric shape around a point in which a first diagonal line connecting a pair of acute angle portions having acute angles opposing to each other, crosses a second diagonal line connecting a pair of obtuse angle portions having obtuse angles opposing to each other;
at least one screw insertion hole provided on the first diagonal line of the main body portion or on the second diagonal line; and
at least one spike provided on each of the first diagonal line and the second diagonal line.

2. The vertebra body plate of claim 1, wherein the spike is formed in an approximately V-shaped in cross section.
